# EUROPÄISCHE PATENTANMELDUNG

(11) **EP 2 143 463 A1**
(43) Veröffentlichungstag der Anmeldung: **13.01.2010**
(21) Anmeldenummer: 09162479.1
(22) Anmeldetag: 11.06.2009
(51) Int. Cl.: A61N 1/05

(54) **Schockelektrodenleitung**

(30) Priorität: 08.07.2008 DE 102008040254
(71) Anmelder: Biotronik CRM Patent AG, 6341 Baar (CH)
(72) Erfinder: Kolberg, Gernot, 12043, Berlin (DE); Bartels, Klaus, 10115, Berlin (DE); Günther, Thomas, 14552, Michendorf OT Wilhelmshorst (DE)
(74) Vertreter: Lindner-Vogt, Karin L.

(57) **Zusammenfassung**

Implantierbare Schockelektrodenleitung mit einem proximalen Ende zur Verbindung mit einem implantierbaren Gerät, welches Schockimpulse erzeugt, und einem distalen Abschnitt, der eine Schockelektrode aufweist, wobei ein Flächenverhältnis der Schockelektrodenfläche zur Oberfläche der Schockelektrodenleitung über die Längserstreckung der Schockelektrode nicht konstant ist.

## Beschreibung

Die Erfindung betrifft eine implantierbare Schockelektrodenleitung zur Versorgung eines biologischen Gewebes und/oder eines Organs mit einer Elektroschocktherapie und eine implantierbare Schockelektrodenanordnung.

Implantierbare Elektrodenleitungen, insbesondere Herzelektrodenleitungen zum endokardialen Abfühlen von Herzaktionspotentialen und/oder zur elektrischen Stimulation oder Defibrillation des Herzen, sind in großer Vielgestaltigkeit bereits bekannt und seit langem in Verbindung mit implantierten Herzschrittmachern oder Defibrillatoren massenhaft im praktischen Einsatz. Endokardiale Systeme (ICDs) reduzieren Morbidität und Mortalität dramatisch und machen das Therapieverfahren zu einer realistischen Option für große Patientengruppen.

Fig. 1 zeigt den Grundaufbau einer implantierbaren Defibrillationsanordnung 1, wie es aus der US 5,531,766 bekannt ist. Um das Herz H eines Patienten P zu stimulieren, steht ein Defibrillator 3 in elektrischer Verbindung mit einer Elektrodenleitung 5, welche mindestens eine Elektrode ("Schockelektrode") 7 trägt, die sich an ihrem distalen Ende befindet und im Herzen des Patienten verlegt wird. Die Defibrillation erfolgt in der Regel über einen Strompfad zwischen dieser Elektrode und einer Gegenelektrode, die mehr oder weniger entfernt von dem zu stimulierenden Herzen liegt. Hierbei spielt die Position und die Struktur der Elektrode bei einer leistungsfähigen Stimulation bzw. Defibrillation eine wesentliche Rolle. Das Gehäuse 8 des Defibrillatiors 3, das die Einheiten zur Detektion von Herzsignalen und Erzeugung von elektrischen Impulsen enthält, kann als Gegenelektrode wirken. Alternativ kann eine Gegenelektrode am Gehäuse außen angebracht sein.

Ähnlich einer Schrittmacherimplantation sieht das ICD-Verfahren den Zugang über die V. cephalica oder V. subclavia und die Positionierung einer Defibrillationssonde im rechten Ventrikel vor. ICD-Sonden sind mit einer einzigen Schockelektrode für die Platzierung im rechten Ventrikel erhältlich, oder sie tragen eine zweite weiter proximal, die typischerweise im rechtem Vorhof, in der V. cava superior oder V. subclavia, positioniert wird.

Grundsätzlich bieten "Hot-can"-Systeme zwei Konfigurationsmöglichkeiten: eine "Single-coil"-Sonde mit dem Schockvektor von rechtsventrikulär zum ICD-Gehäuse (als unipolar bezeichnet) und eine "Dual-coil"-Sonde mit dem Defibrillationsfeld zwischen rechtem Ventrikel, V. cava superior und Gehäuse ("Triad"-Konfiguration).

Aus der US 5 203 348 ist eine Defibrillationselektrode für eine subkutane Applikation in Form einer großflächig ausgebildeten Spirale bekannt, welche das distale Ende der Zuleitung der Defibrillationselektrode bildet. Die wendelartige Form wird hierbei dazu genutzt, um die effektive Elektrodenfläche zu erhöhen.

Die wendelartigen Schockelektroden werden aus einem oder mehreren parallelen Drähten, Bändern oder kleinen Wendeln gewickelt. Hierbei ist der elektrische Widerstand über die Draht- bzw. Bandlänge konstant.

Da die Stromdichteverteilung einer wendelartigen Elektrode abhängig von der elektrisch aktiven Oberfläche, dem elektrischen Widerstand des Wendelmaterials und, abgesehen von den Körperstrukturen des Patienten, von der Lage der Elektroden zueinander ist, kann die Stromdichteverteilung mit herkömmlichen Konstruktionen nur eingeschränkt beeinflusst werden. Außerdem produziert ein Elektroschock, der zum Herzen oder Thorax abgegeben wird, kein perfekt uniformes elektrisches Feld. In einem sogenannten "Dualcoil-System" liegen die Schockwendeln zum Beispiel eher hintereinander als achsparallel, so dass die Gewebe bzw. die Organe, die zwischen den Elektroden liegen, einer ungleichmäßigen Feldverteilung ausgesetzt werden. Das Myokard in Regionen höherer Feldstärke (typischerweise nahe der Defibrillationselektrode) wird deshalb größere Effekte bei De- und Repolarisation aufweisen als Zellen im schwächeren elektrischen Feld. Diese bekannte Variabilität der zellulären Antwort in einem inhomogenen Schockfeld wird als "graded response" bezeichnet.

In einer Defibrillationstherapie ist die Defibrillationsschwelle (DFT) ein bedeutsamer Faktor, der in Betracht gezogen werden muss. Die Defibrillationsschwelle entspricht der niedrigsten Schockenergie, mit der tatsächlich das Kammerflimmern beendet wird. Da die Defibrillationsschwelle von den "zuletzt" erreichten Herzzellen abhängt, kann davon ausgegangen werden, dass bei ungleichmäßiger Feldverteilung gewisse Gewebezonen mit unnötig viel Energie versorgt werden. Bei einer ausgewogeneren Feldverteilung kann dagegen die DFT gesenkt werden.

Eine Beeinflussung des elektrischen Feldes kann dadurch vorgenommen werden, dass die Polarität der Schockelektroden gewechselt wird, wie in US 6,449,506 offenbart ist. Hierdurch kann jedoch die Übergangsimpedanz zum Elektrolyten nur in geringen Grenzen eingestellt werden. Unter Übergangsimpedanz zum Elektrolyten versteht man hierbei den Gesamtwiderstand des Strompfades zwischen der Schockelektrode und der Gegenelektrode über ein elektrolytisches Material (Gewebe, Organ, Blut).

Es ist daher die Aufgabe der Erfindung, eine verbesserte implantierbare Schockelektrodenleitung bzw. eine entsprechende Schockelektrodenanordnung anzugeben, die bei gleicher Wirksamkeit die Anwendung einer reduzierten Schockenergie ermöglicht.

Diese Aufgabe wird gemäß einem ersten Aspekt der Erfindung durch eine implantierbare Schockelektrodenleitung mit den Merkmalen des Anspruchs 1 und gemäß einem zweiten Aspekt der Erfindung durch eine implantierbare Schockelektrodenanordnung mit den Merkmalen des Anspruchs 13 gelöst. Zweckmäßige Fortbildungen des Erfindungsgedankens sind Gegenstand der abhängigen Ansprüche.

Die erfindungsgemäße Schockelektrodenleitung zeichnet sich dadurch aus, dass ein Flächenverhältnis der Schockelektrodenfläche zur Oberfläche der Schockelektrodenleitung über die Längserstreckung der Schockelektrode nicht konstant ist. Dies bietet einen großen Spielraum bei der Gestaltung von Schockelektrodenleitungen. Die mechanischen Eigenschaften sowie der elektrische Widerstand der Schockelektrode und die Übergangsimpedanz zum Elektrolyten können hierdurch über die Länge der Schockelektrode gezielt eingestellt werden. Damit können einerseits die Stromdichteverteilung beispielsweise beim ICD-Schock, andererseits die mechanischen Eigenschaften der Schockelektrode gezielt beeinflusst werden. Die Gestaltungsmöglichkeiten können eingesetzt werden, um die Defibrillationsschwelle zu senken, um das Einwachsverhalten zu beeinflussen, um dem Perforationsrisiko vorzubeugen und um die Dauerfestigkeit der Schockelektrode zu erhöhen. Außerdem kann hierdurch die Steifigkeit der Schockelektrode über ihre Länge gezielt beeinflusst werden, wodurch die mechanischen Reizungen, zum Beispiel im Herzen, minimiert werden können.

In einer Gestaltung, in der das distale Ende der Schockelektrode am weitesten von der Gegenelektrode entfernt ist, ist die Übergangsimpedanz zum Elektrolyten bzw. der Gesamtwiderstand über den resultierenden Strompfad am höchsten. Daraus folgt, dass die Gewebezonen an dieser Stelle einem relativ niederenergetischen Schock ausgesetzt werden. Eine bevorzugte Ausführung der Erfindung besteht darin, dass das Flächenverhältnis der Schockelektrodenfläche zum distalen Ende der Schockelektrode hin zunimmt. Hierdurch wird die Stromstärke am distalen Ende der Elektrode und somit auch die eingetragene Schockenergie an den dort angrenzenden Gewebezonen erhöht. Durch diese Maßnahme wird eine ausgewogenere Feldverteilung erreicht, die in einem Defibrillationsprozess zu einer Senkung der Defibrillationsschwelle führt.

Alternativ kann das Flächenverhältnis der Schockelektrodenfläche zum distalen Ende der Schockelektrode hin abnehmen oder andersartig über die Länge der Elektrode variieren, je nachdem, wie die Gesamtkonfiguration der Kardioversionsanordnung aussehen soll und in welcher konkreten Weise man das elektrische Feld an verschiedenen Stellen der Schockelektrode variieren möchte.

Um das von der Schockelektrode erzeugte elektrische Feld weiter zu beeinflussen, kann der Strom mindestens teilweise direkt zum distalen Ende der Elektrode hin geleitet werden. Der Reihenwiderstand des Schockelektrodeabschnittes am distalen Ende ist somit niedriger als der Reihenwiderstand des übrigen Abschnittes. Da die Übergangsimpedanz zum Elektrolyten direkt von dem inneren Elektrodenwiderstand abhängt, ermöglicht die direkte Stromeinspeisung am distalen Ende der Elektrode dort eine Senkung der Übergangsimpedanz zum Elektrolyten.

Eine technologisch zweckmäßige Ausführungsform sieht eine Schockelektrode vor, die als Rohr ausgebildet ist. Das Rohr, das eine stabilere Struktur der Elektrode gewährleistet, weist einen Kern aus isolierendem Material (Silikon oder ähnliches Polymer) auf. Auf der Oberfläche dieses Rohrs ist eine leitfähige Struktur angeordnet, welche der elektrischen aktiven Schockelektrodenfläche entspricht und direkt in Kontakt mit dem zu stimulierendem Gewebe bzw. Organ steht.

Die leitfähige Struktur ist bevorzugt als mehrschichtige Struktur ausgebildet. Sie weist mindestens zwei Schichten mit unterschiedlicher Leitfähigkeit auf. Die auf der Oberfläche des Rohrs angeordnete erste Schicht kann aus mechanisch stabilem und elektrisch gut leitfähigem Tantal bestehen, während die zweite Schicht bzw. das Deckmaterial aus gut körperverträglichem Platin oder aus einer Platin-Iridium-Legierung besteht.

Alternativ kann die leitfähige Struktur auf einer Schicht aus Formgedächtnislegierung (Memorymetall), die das ganze Rohr beschichtet, angeordnet werden. Solche Legierungen, wie zum Beispiel Nickel-Titan-Legierungen (Nitinol), können sich an eine frühere Formgebung trotzt nachfolgender starker Verformung scheinbar "erinnern". Dies ist besonders nützlich, wenn man nach der Implantation die Form der Elektrode ändern will.

Die mehrschichtige Struktur kann bereits beim Ziehen des Rohres gebildet werden. Mehrere Rohre aus verschiedenen Werkstoffen werden ineinander gesteckt und auf den gewünschten Durchmesser gezogen. Alternativ können die Schichten durch einen galvanischen Prozess bzw. durch Bedampfen oder durch Sputtern auf einem Kernrohr ausgebildet werden.

Die leitfähige Struktur kann dann durch einen Laserschneid- oder Ätzprozess verschiedene Formen annehmen. Mit den geschnittenen Strukturen lassen sich die mechanischen Eigenschaften (Biege- und Torsionssteifigkeit, Zug- und Druckspannung), die elektrischen Eigenschaften (Widerstand) und die Ankoppelung an den Elektrolyten (Kontaktfläche) über die Länge der Schockelektrode variieren.

Wenn das Kernrohr mit einem leitfähigen Material komplett beschichtet ist, sind die Abstände zwischen den geschnittenen leitfähigen Strukturen bevorzugt mit einem isolierenden Material wie Silikongummi ausgefüllt, so dass nur das Deckmaterial der mehrschichtigen Struktur in elektrischen Kontakt mit dem Gewebe und/oder Organ steht.

In einer weiteren Ausführung der Erfindung ist vorgesehen, dass die leitfähige Struktur parallele Ringe mit unterschiedlicher Breite aufweist. Die Breite der Ringe kann an verschiedenen Stellen der Schockelektrode unterschiedlich sein. Sie kann zum Beispiel zum distalen Ende der Elektrode hin zunehmen bzw. abnehmen. Um die parallelen Ringe elektrisch zu verbinden, sind zwischen den Ringen leitfähigen Stege vorhanden.

In einer alternativen Ausführungsform weist die leitfähige Struktur sich längs der Elektrode erstreckende Streifen mit nicht konstanter Breite auf. Diese Streifen können zum Beispiel zum distalen Ende der Elektrode hin enger bzw. breiter werden.

Eine weitere alternative Ausführung sieht eine leitfähige Struktur vor, die eine Wendel aufweist, deren Steigung nicht konstant ist. Die Steigung kann zum Beispiel zum distalen Ende der Elektrode hin zunehmen bzw. abnehmen. In ähnlicher Weise kann die leitfähige Struktur eine Netz- bzw. Gitterstruktur aufweisen, die mindestens zwei ineinander verflochtene Wendelstrukturen mit nicht konstanter Steigung aufweist.

Neben der Wendelstruktur sind auch axiale Strukturelemente denkbar wie zum Beispiel Querverbindungen zwischen den Windungen oder zwischen andersartigen Flächen (Scheiben, Ringe). In einer Ausführung der Erfindung können diese Strukturelemente in ihre Größe über die Länge der Schockelektrode variieren, wobei die Steigung der Wendel konstant bleibt oder ebenfalls variiert.

Eine weitere Ausführung sieht eine leitfähige Struktur vor, die Schichten mit einer nicht konstanten Höhe längs der Elektrode aufweist. Die Schichtenhöhe kann an verschiedenen Stellen der Schockelektrode unterschiedlich sein, so dass unterschiedliche Härten längs der Elektrode vorgesehen sind, um z. B. eine oder mehrere Vorzugsbiegerichtungen zu erhalten.

Selbstverständlich sind die oben beschriebenen Ausführungsformen der leitfähigen Struktur auf einer Schockelektrode miteinander kombinierbar.

In einer etwas modifizierten Ausführung der Erfindung ist die Schockelektrode als Drahtwendel (Schockelektrodenwendel) ausgebildet, deren Steigung nicht konstant ist. Hierbei kann die Wendel zum distalen Ende der Schockelektrode hin eng bzw. weit gewickelt werden. Die Steigung kann selbstverständlich andersartig über die Länge der Elektrode variieren, je nachdem, ob man das elektrische Feld an verschiedenen bzw. unterschiedlichen Stellen der Schockelektrode variieren möchte.

Eine erfindungsgemäße implantierbare Schockelektrodenanordnung zeichnet sich dadurch aus, dass eine der oben beschriebenen Schockelektrodenleitungen am proximalen Ende in Verbindung mit einem implantierbaren Gerät, z. B. einem Defibrillator, steht. Das Gerät wirkt als Gegenelektrode und weist ein Gehäuse auf, welches in der Regel eine Steuereinheit zum Abfühlen bzw. zur Erzeugung elektrischen Impulse und eine Batterie zur Energieversorgung enthält.

Im Besonderen hat die Schockelektrodenleitung zusätzlich einen Sensor eine Abfühlelektrode zum Abfühlen von Gewebe- und/oder Organaktionspotentialen und eine Stimulationselektrode zur niederenergetischen elektrischen Stimulation.

Abfühl- und Stimulationselektrode sind in eine transvenöse Sonde integriert, welche einen zwiebelschalenartigen schichtförmigen Aufbau besitzt (koaxiale Konfiguration). Dabei sind konzentrisch von innen nach außen spiralförmige Leiter für die Detektion/Stimulation und ggf. für die Defibrillation angeordnet. Zwischen den einzelnen Leitern befindet sich jeweils eine eigene Isolationsschicht. Vorteilhaft kann auch ein mehrlumiger Aufbau angewendet sein. Hier sind die Leiter für die Detektion, Stimulation und ggf. Defibrillation parallel innerhalb eines Elektrodenkörpers angeordnet und werden jeweils von einer eigenen Isolationsschicht umhüllt. Zur Stabilisierung sind im Elektrodenkörper zusätzlich längs angeordnete Hohlräume enthalten. Vorteile mehrlumiger Elektroden sind ein geringerer Durchmesser und eine bessere Langzeitstabilität.

Im Hinblick auf die seit langem etablierten implantierbaren Schockelektrodenanordnungen ist es von Vorteil, dass die erfindungsgemäße Anordnung eine ausgewogenere Feldverteilung über die zu stimulierenden Gewebe- bzw. Organzonen ermöglicht. In einem ICD-System kann dies zu einer Senkung der Defibrillationsschwelle führen. Daraus folgt, dass die Gewebe bzw. Organe einer unnötigen und eventuell schädlich hohen Energie nicht ausgesetzt werden und dass die im implantierbaren Gerät vorhandene Batterie eine längere Lebensdauer aufweist.

Vorteile und Zweckmäßigkeiten der Erfindung ergeben sich im Übrigen aus der nachfolgenden Beschreibung ausgewählter Ausführungsbeispiele anhand der Figuren. Von diesen zeigen:

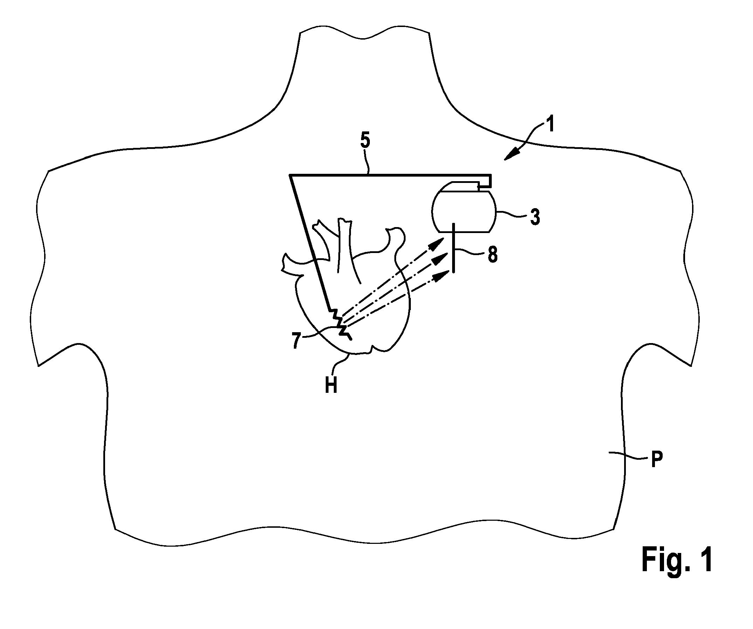

- Fig. 1: eine schematische Darstellung einer ICD-Anordnung,
- Fig. 2: eine schematische Darstellung einer implantierbaren Schockelektro- denleitung gemäß der Erfindung,
- Fig. 3A und 3B: schematische Darstellungen eines Abschnittes der Schockelektrode gemäß einer Ausführungsform der Erfindung, und
- Fig. 4A bis 4D: schematische Darstellungen des distalen Abschnittes der Schockelekt- rodeleitung gemäß alternativen Ausführungsformen der Erfindung.

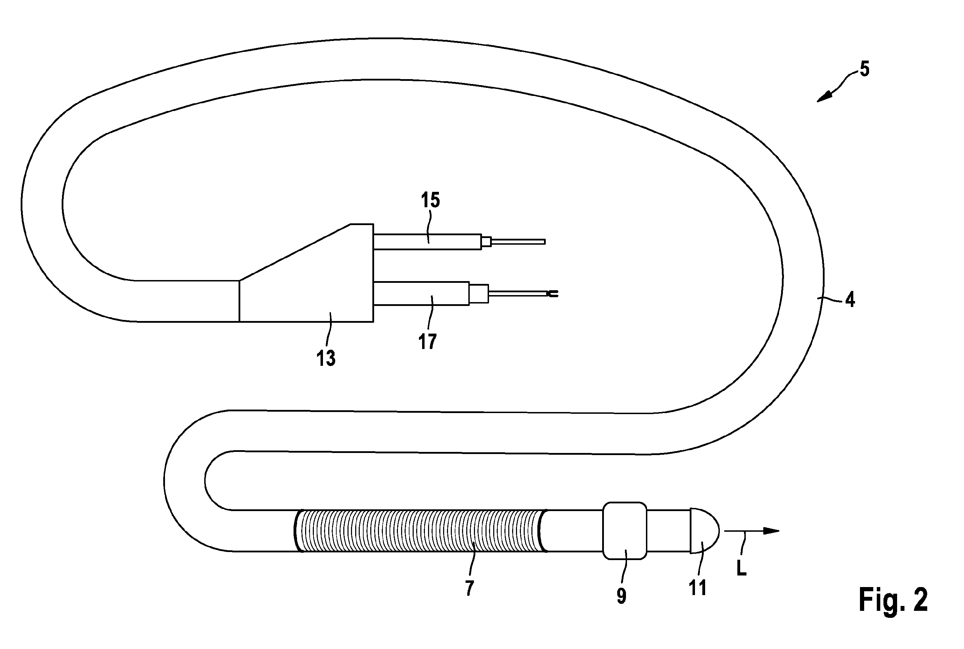

Fig. 2 zeigt eine implantierbare Schockelektrodenleitung 5, die einen flexiblen Kunststoffschlauch 4 aufweist, welcher ein proximales Ende und einen distalen Abschnitt hat. Im distalen Abschnitt weist die Schockelektrodenleitung 5 eine langgestreckte Schockelektrode 7 auf, die in Wandkontakt im Herzen eines Patienten verlegt wird, und eine Ring-Elektrode 9 und eine Tip-Elektrode 11, die für Abtast- und/oder Stimulationszwecke vorgesehen sind. Am proximalen Ende sind aus einem Y-Verteiler 13 sich erstreckende Steckerelemente 15, 17 vorgesehen, die der Schockelektrode 7 und den Stimulations- bzw. Detektionselektroden 9, 11 zugeordnet sind und die in elektrischer Verbindung mit einem (nicht dargestellten) implantierbaren Gerät stehen.

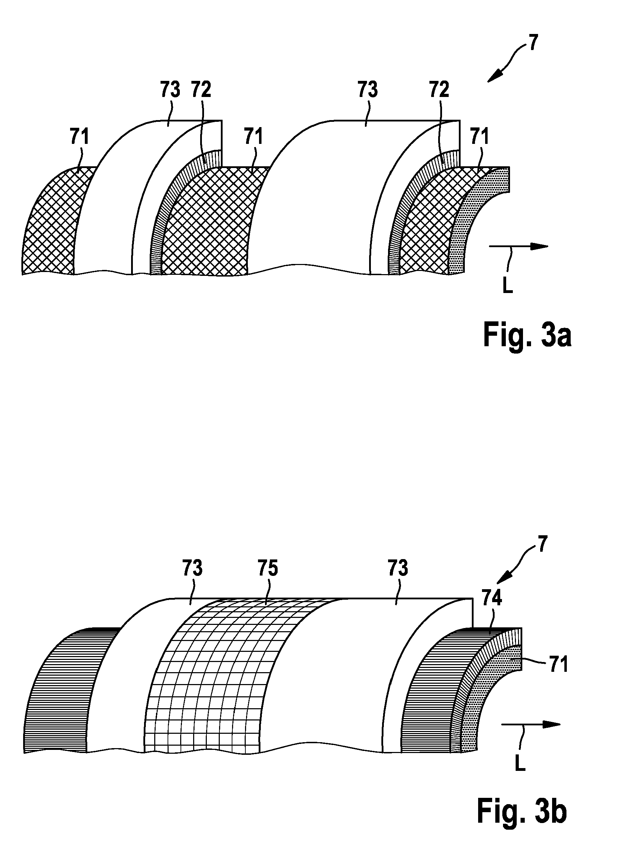

Fig. 3A zeigt einen Abschnitt der Schockelektrode 7, die als Rohr ausgebildet ist. Auf dem Kernrohr 71 ist eine zweischichtige leitfähige Struktur angeordnet. Die erste Schicht 72, die in direktem Kontakt mit dem Kernrohr 71 steht, besteht aus einem höher leitfähigen Material im Vergleich mit dem Material einer zweiten Schicht 73, welche die erste Schicht 72 bedeckt, aber besser körperverträglich als jene ist.

Fig. 3B zeigt eine Variante der Ausführung der Fig. 3A. Die leitfähige Struktur, die hier nur von der Deckschicht 73 gebildet wird, ist auf einer Schicht von Formgedächtnislegierung 74 angeordnet, die das Kernrohr 71 beschichtet. Hierbei sind die Abstände zwischen den leitfähigen Strukturen mit einem isolierenden Material 75 ausgefüllt, so dass die Deckschicht 73 der elektrischen aktiven Schockelektrodenfläche, die in Kontakt mit dem Gewebe und/oder Organ steht, entspricht.

Aus den beiden Figuren 3A und 3B ist jeweils klar ersichtlich, dass sich die Breite der aktiven Schockelektrodenfläche über die Länge L der Elektrode ändert.

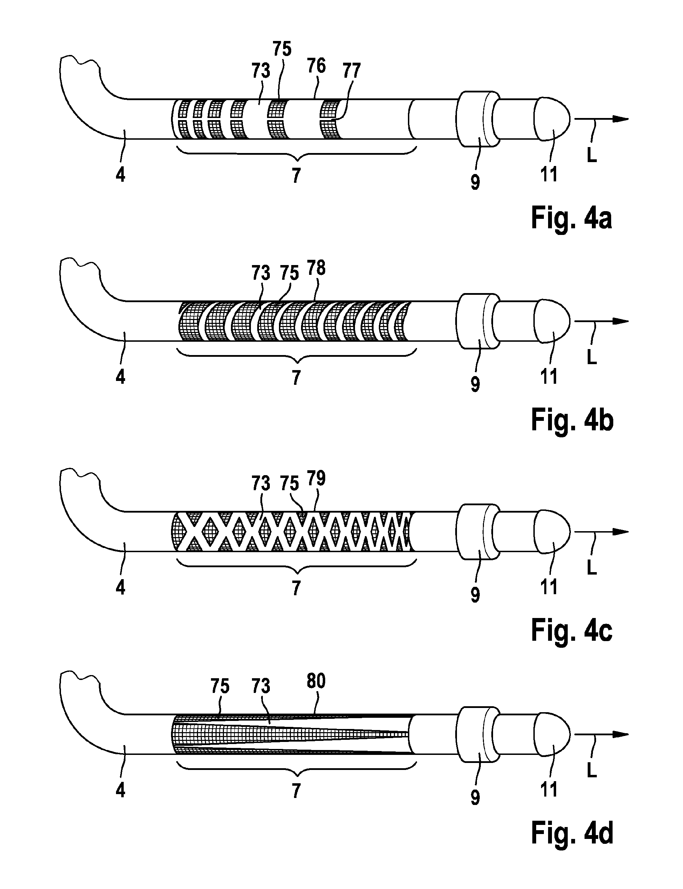

Fig. 4A bis Fig. 4D zeigen mögliche Ausführungsformen der Schockelektrode 3, die als Rohr ausgebildet ist.

Fig. 4A zeigt den distalen Abschnitt der Schockelektrodenleitung 5, wobei die leitfähige Struktur parallele Ringe 76 mit unterschiedlicher Breite aufweist, die durch leitfähige Stege 77 verbunden sind. Die Breite der Ringe nimmt zum distalen Ende der Elektrode hin zu.

Fig. 4B zeigt den distalen Abschnitt einer Schockelektrodenleitung 5', bei der die leitfähige Struktur eine Wendel 78 aufweist. Die Steigung der Wendel nimmt zum distalen Ende der Elektrode hin zu.

In ähnlicher Weise zeigt Fig. 4C den distalen Abschnitt einer Schockelektrodenleitung 5" gemäß einer weiteren Ausführung der Erfindung, wobei die leitfähige Struktur eine Netzstruktur 79 aufweist, welche zum distalen Ende der Elektrode hin dichter wird.

Fig. 4D schließlich zeigt als weitere Ausführung eine Schockelektrodenleistung 53', bei der als leitfähige Oberflächenstruktur längs verlaufende Streifen 18 mit zum distalen Ende hinzunehmender Breite vorgesehen sind.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Schockelektrodenleitung (5) mit einem proximalen Ende zur Verbindung mit einem implantierbaren Gerät (3), welches Schockimpulse erzeugt, und einem distalen Abschnitt, der eine Schockelektrode (7) aufweist, wobei ein Flächenverhältnis der Schockelektrodenfläche zur Oberfläche der Schockelektrodenleitung (5) über die Längserstreckung der Schockelektrode (7) nicht konstant ist.

2. Schockelektrodenleitung (5) nach Anspruch 1, wobei das Flächenverhältnis zum distalen Ende der Schockelektrode (7) hin zunimmt.

3. Schockelektrodenleitung nach einem der vorhergehenden Ansprüche, wobei die Schockelektrode (7) als Rohr mit einer auf seiner Oberfläche angeordneten leitfähigen Struktur ausgebildet ist.

4. Schockelektrodenleitung (5) nach Anspruch 3, wobei die leitfähige Struktur parallele Ringe (76) mit unterschiedlicher Breite aufweist.

5. Schockelektrodenleitung (5) nach Anspruch 3 oder 4, wobei die leitfähige Struktur sich längs der Schockelektrode (7) erstreckende Streifen mit nicht konstanter Breite aufweist.

6. Schockelektrodenleitung nach einem der Ansprüche 3 bis 5, wobei die leitfähige Struktur eine Wendel (78) aufweist, deren Steigung nicht konstant ist.

7. Schockelektrodenleitung (5) nach einem der Ansprüche 3 bis 6, wobei die leitfähige Struktur eine Netzstruktur (79) aufweist, die mindestens zwei ineinander verflochtene Wendelstrukturen mit nicht konstanter Steigung und/oder Ringe und/oder Streifen mit nicht konstanter Breite aufweist.

8. Schockelektrodenleitung (5) nach einem der Ansprüche 3 bis 7, wobei die leitfähige Struktur eine nicht konstante Höhe längs der Schockelektrode (7) aufweist, um eine Vorzugsbiegerichtung der Schockelektrode (7) zu erhalten.

9. Schockelektrodenleitung (5) nach einem der Ansprüche 3 bis 8, wobei die leitfähige Struktur mindestens zwei Schichten (72, 73) mit unterschiedlicher Leitfähigkeit aufweist.

10. Schockelektrodenleitung (5) nach einem der Ansprüche 3 bis 9, wobei die leitfähige Struktur auf einer Schicht von Formgedächtnislegierung (74) angeordnet ist.

11. Schockelektrodenleitung (5) nach einem der Ansprüche 3 bis 10, wobei die leitfähige Struktur durch einen Laserschneidprozess ausgebildet ist.

12. Schockelektrodenleitung (5) nach Anspruch 1, wobei die Schockelektrode (7) als Drahtwendel ausgebildet ist, deren Steigung nicht konstant ist.

13. Implantierbare Schockelektrodenanordnung (1), umfassend eine Schockelektrodenleitung (5) nach einem der Ansprüche 1 bis 12 und ein implantierbares Gerät (3) mit einem Gehäuse, das als Gegenelektrode wirkt.

14. Schockelektrodenanordnung (1) nach Anspruch 13, wobei die Schockelektrodenleitung (1) einen Sensor (9) zum Abfühlen von Gewebe- und/oder Organaktionspotentialen und eine Stimulationselektrode (11) zur niederenergetischen elektrischen Stimulation aufweist.
